# EUROPEAN PATENT SPECIFICATION

(11) **EP 2 170 103 B1**
(45) Date of publication and mention of the grant of the patent: **28.12.2011**
(21) Application number: 08785226.5
(22) Date of filing: 30.07.2008
(51) Int. Cl.: A23L 1/29, A23L 1/30, A61K 31/20

(54) **CACHEXIA PREVENTION SUPPLEMENT**
NAHRUNGSERGÄNZUNG ZUR VERHINDERUNG VON ABMAGERUNGEN
COMPLÉMENT ALIMENTAIRE DE PRÉVENTION DE LA CACHEXIE

(30) Priority: 31.07.2007 EP 07015027
(43) Date of publication of application: 07.04.2010
(73) Proprietor: Fresenius Kabi Deutschland GmbH, 61352 Bad Homburg v.d.H. (DE)
(72) Inventor: SCHNEID, Christina, 55118 Mainz (DE)
(74) Representative: Richly, Erik
(86) International application number: PCT/EP2008/006281
(87) International publication number: WO 2009/015879

(56) References cited:
- WO-A-00/07607
- WO-A-2004/105517
- WO-A-2005/110124
- WO-A-2007/149590
- DE-U1-202005 012 984
- DE-U1-202006 014 588
- DE-U1-202007 000 949
- US-A1- 2005 032 757
- US-B1- 6 649 195
- MANTOVANI G ET AL: "CANCER-RELATED ANOREXIA/CACHEXIA SYNDROME AND OXIDATIVE STRESS: AN INNOVATIVE APPROACH BEYOND CURRENT TREATMENT" CANCER EPIDEMIOLOGY, BIOMARKERS AND PREVENTION, PHILADELPHIA, PA, vol. 13, no. 10, 1 October 2004 (2004-10-01), pages 1651-1659, XP008065515 ISSN: 1055-9965

## Description

The present invention relates to a nutritional composition, preferably a nutritional supplement, for the prevention and/or treatment of cachexia and/or anorexia. The composition according to the present invention contains as constituents eicosapentaenoic acid or a derivative thereof and at least one phytochemical. In a preferred embodiment, the composition additionally contains one or more branched-chain amino acids and/or glutamine, both in neutral or salt form or a precursor or derivative thereof. In a particularly preferred embodiment the composition additionally comprises one or more further antioxidants such as mixed carotenoids, vitamin E, vitamin C, zinc, magnesium and selenium.

### Background Art

Cachexia represents progressive wasting of muscle and adipose tissue and is associated with increased morbidity and mortality. The term derives from the Greek kakos, which means "bad", and from hexis meaning "condition", and is generally understood to refer to a state of ill health and malnutrition. It is often associated with and induced by an underlying disease e.g. malignant cancer, and its clinical syndrome is characterized by loss of appetite (particularly anorexia), loss of body weight accompanied by a decrease in muscle mass and adipose tissue, tissue wasting, fatigue, and a poor performance status that often precedes death.

Multiple mechanisms appear to be involved in the development of cachexia, including anorexia, decreased physical activity, decreased secretion of host anabolic hormones, and altered host metabolic response with abnormalities in protein, lipid and carbohydrate metabolism.

Although anorexia, one of the main features of the cachexia syndrome usually accompanies cachexia, cachexia rarely responds to increased food intake alone. The pathogenesis of anorexia is most certainly multifactoral but is not yet well understood. It seems to be attributable in part to intermediary metabolites like lactate ketons and oligonucleotides or to other substances released such as acute phase response proteins.

An increased resting energy expenditure may contribute to the loss of body weight in cachectic patients and may explain the increased oxidation of fat tissue. Futile energy-consuming cycles, such as the Cori-cycle, may also play a role in the increased energy demand.

Body weight loss in cachectic patients arises equally from loss of muscle and fat, characterized by increased catabolism of skeletal muscle and decreased protein synthesis. Catabolic factors capable of direct breakdown of muscle and adipose tissue appear to be secreted in cachexia and may play an active part in tissue degeneration.

Cachexia is related to underlying diseases as e.g. cancer, chronic obstructive pulmonary disease (COPD), chronic heart failure, HIV/Aids, chronic kidney disease, neurological disease, and rheumatic disease. The frequency of cachexia in the underlying disease could be estimated with e.g. 50 % of all cancer patients, 30 % of all COPD patients and 10 % of all chronic heart failure patients.

The present knowledge of the underlying mechanisms responsible for cachexia remains incomplete. However, most states of cachexia are associated with underlying inflammatory processes. The mechanisms of muscle protein reduction are considered to be as follows. During the phenomena of cachexia, progressive reduction of skeletal muscle mass occurs, although visceral protein reserves are preserved and the liver mass may actually increase. Whole-body protein turn-over is increased, due to an increase in muscle protein catabolism and an overall decrease in protein synthesis, despite the increase in hepatic acute-phase protein synthesis.

There are three main proteolytic pathways responsible for protein catabolism in skeletal muscle. These are the lysosomal system, which is involved in proteolysis of extracellular proteins and cell-surface receptors, the cytosolic calcium-regulated calpains, which are involved in tissue injury, necrosis and auteolysis, and the ATP ubiquitin-dependent proteolytic pathway. Of these. ubiquitin-dependent proteolysis is considered to be the most important for protein degradation in (cancer) cachexia.

The mechanisms of adipose tissue reduction are understood to be as follows. Cachexia is also characterized by a marked reduction in adipose tissue, which is due to an increase in lipolysis rather than a decrease in lipogenesis. In addition, energy metabolism is dysregulated during the cachectic state.

Activation of the pathogenic mechanisms of cachexia is an early phenomenon, and cachexia should be suspected even in the absence of weight loss, particularly in those patients whose disease prognosis are known to negatively influence nutritional status. In the early phase of this development processes of metabolic/nutritional imbalance might already be active before signs of tissue loss become clinically apparent.

The onset of anorexia-cachexia significantly influences the clinical course of the disease, and most therapies actually exacerbate anorexia and worsen body weight loss. As a consequence, the higher prevalence and greater severity of anorexia-cachexia syndrome in advanced cancer patients is mostly due to iatrogenic cause. The presence of early satiety at any stage of the disease can significantly increase the risk of death by 30%.

An interesting aspect of the clinical impact of anorexia-cachexia syndrome that has recently emerged is its influence on quality of life. Supporting this view, it was recently documented that quality-of-life function scores are largely determined by nutrient intake and weight loss, accounting for 20% and 30% of the total score respectively.

As pointed out above, the most common requirements for cachexia are the presence of an underlying disease such as an inflammatory process or the presence of malignant cancer tissue. Numerous etiologic circulating factors have been reported in patients with cachexia syndrome. Evidence from both animal and human studies suggest that a number of pro-inflammatory cytokines play etiologic roles in the development of cachexia. Interleukin-6 (IL-6), tumor necrosis factor-α (TNF-α), interleukin-1 (IL-1), and interferon-γ (IFN-γ) are the cytokines implicated in most studies, especially cachexia associated with cancer and critical illness. In addition, tumor-derived compounds such as lipid-mobilizing factor, anemia-inducing substrates and proteolysis-inducing factors have also been implicated in cachexia. Overall, cachexia results form the interplay between multiple cytokines, neuropeptides, hormones and tumor or pathogen-related compounds that interact in such a manner as to alter cellular metabolism and result in tissue loss.

In summary the causes of cachexia are multiple and differ, at least in part in different diseases, but some of them are common and they constitute the main background on which the symptoms of cachexia are based. Among the common causes of cachexia the most well-recognized are:
■ Proinflammatory cytokines with related symptoms
■ Hypermetabolism
■ Neurotransmitter
■ Hormone changes
■ Anorexia.

The treatment of cachexia was up to now ineffective using for example structured lipids under enteral or parenteral application. Moreover, the use of fish-oil capsules is questionable and had experienced serious draw-backs including swallowing, eructation and bad taste.

EP 0 914 111 describes cachexia and anorexia treating compositions comprising an oil blend containing ω-6 fatty acids and ω-3 fatty acids, together with a source of amino-nitrogen comprising branched-chain amino acids and further antioxidant components. EP 0 914 111 is silent about any phytochemicals.

WO 2004/026294 relates to a leucine-enriched nutritional composition, which may contain ω-3 polyunsaturated fatty acids. The composition is used for promoting muscle protein synthesis or controlling tumor-induced weight loss. However, WO 2004/026294 is silent about the presence of phytochemicals.

WO 00/07607 relates to a nutrient and therapeutic composition for the treatment of cancer which may contain a wide variety of components in broad ranges. Moreover, there are described several diseases and conditions in connection with cancer apart from cachexia.

Also enteral nutrition with relatively low ω-6:ω-3 (2:1-3:1) were not effective, in most cases no or very low eicosapentaeonic acid (EPA) concentrations were included in the treatment. Also the combination of EPA with nutrient dense oral nutritional supplements is only partly effective due to a lack of synergistic acting amino acids and antioxidants. Moreover, products and therapeutic approaches today tackle only the end-stage of cachexia and not pre-cachexia. Thus, up to now the prevention and treatment of cachexia remains difficult. Both animal and human studies suggest that nutritional support is largely ineffective by the means available today. Especially restoring body stores - as it is lean body mass - is very difficult when the cachectic state is advanced. Given these facts cachexia should be treated in its preclinical stage, when the likelihood for effective prevention of muscle and tissue wasting is greater than during the advanced stage of the disease. Interventions at this early, pre-cachectic phase targeting both cachexia and anorexia seem to be an important and promising therapeutic concept. Once tissue loss is apparent interventional approaches to re-build muscle and fat mass have been proven difficult and with limited success.

In view of the above description of the prior art there is still existing a strong demand to provide for compositions treating cachexia and/or anorexia in a very early and pre-clinical stage, when the likelihood for effective prevention of muscle and tissue wasting is greater than during the advanced stage of the disease.

Therefore, the object underlying the present invention is the provision of a nutritional composition for the prevention and treatment of cachexia and/or anorexia being effective already in the pre-clinical stage. In particular, it is an object to provide for a composition that prevents not only the onset of cachexia and/or anorexia, but also the progression of cachexia/anorexia. Furthermore, it is an object to provide for such a composition which is effective also in the treatment of existing cachexia/anorexia.

### Summary of the Invention

In order to deal with the above-mentioned objects, the present invention provides for a nutritional composition comprising from 0.1 to 10 g of eicosapentaenoic acid or a derivative thereof based on 100 kcal of the total composition, from 0.1 to 10 g of a phytochemical or a mixture thereof based on 100 kcal of the total composition, from 0.1 to 50 g, more preferably from 1 to 25 g based on 100 kcal of the total composition of one or more branched-chain amino acids (BCAA) selected from the group consisting of leucine (Leu), isoleucine (Ile), and valine (Val), in neutral or salt form or a precursor or derivative thereof, from 0.25 to 50 g of glutamine in neutral or salt form or a precursor or derivative thereof based on 100 kcal of the total composition_{,} and in a preferred embodiment the nutritional composition further comprises one or more antioxidants selected from the group consisting of mixed carotenoids, vitamin E, vitamin C, zinc, magnesium, and selenium.

The compositions according to the present invention are for use as a medicament, preferably as a nutritional supplement, to be employed in the prevention and/or treatment of cachexia and/or anorexia even in a pre-clinical stage of the diseases, that means already for preventing the onset of said diseases but also the progression thereof. Furthermore, they are also suitable to be employed in the treatment of existing cachexia/anorexia.

### Detailed Description of the Invention

The present invention provides for a nutritional composition for the prevention and/or treatment of cachexia and/or anorexia. According to the invention, the composition is intended to comprise nutritional supplement compositions (nutritional supplements) and complete foodstuff compositions. In a preferred embodiment, the compositions according to the invention the composition is a nutritional supplement, which is administered to the patient two times per day. However, even if the administration two times per day is preferable, the composition may also be administered only once per day or e.g. up to five times per day.

In case of a nutritional supplement composition to be employed in the prevention of cachexia and/or anorexia, it is particularly preferable to limit the caloric value of the composition to a specific value in order to minimize the impact of the supplement on the total diet of the patient. In other words, as the composition according to the present invention is intended to provide for a preventive effect on cachexia and/or anorexia and, therefore, is administered to persons not showing any nutritional deficiency symptom, the caloric value is kept as low as possible and should not exceed 400 kcal per daily dose, preferably not more than 300 kcal per daily dose, and more preferably not more than 200 kcal per daily dose. The same is true in case the composition according to the invention is used as a nutritional supplement in the treatment of cachexia and/or anorexia, when supplemented to other enteral or parenteral nutritional compositions. Generally, it is important to note that according to the invention the amounts of the constituents (such as EPA, the phytochemical, and, if present, BCAA, glutamine etc.) of the composition are remarkably high taking the desired low total caloric value of the compositions as mentioned above into account. Preferably, the caloric value is basically provided by EPA, BCAA and glutamine.

Therefore, the amounts of the components of the composition are indicated hereinbelow in the unit "g/kcal". Alternatively, the amounts of the constituents are indicated hereinbelow as "g/daily dose". These values may be easily re-calculated into "wt-%" based on the fact that a particularly preferred composition according to the present invention provides for a caloric value of about 100 kcal (Example 1). The general description hereinbelow is intended to apply for all compositions of the invention independently from the indicated unit.

In the following the components of the nutritional composition according to the present invention will be described in more detail.

As a first essential component the composition according to the present invention comprises eicosapentaenoic acid (EPA) which belongs to the group of ω-3 polyunsaturated fatty acids. Without being bound by any theory, the use of EPA is suggested for the treatment or prevention of cachexia by inhibiting inflammatory processes, lypolytic activities and the inhibition of the ATP-ubiquitin-pathway. EPA has been shown to suppress the production of pro-inflammatory cytokines and arachidonic mediators. These in vitro effects have been exploited in the treatment of cancer patients, and it has been shown that adequate and prolonged EPA supplementation leads to increased body weight and gain of lean body mass in cachectic cancer patients, probably due to suppression of either cytokine production or pro-inflammatory mediators. In addition, the use of an EPA-enriched, energy-dense oral supplement increases the physical activity which may reflect improved quality of life.

According to the present invention EPA has been found to be particularly effective as a so-called "anti-wasting" factor. An "anti-wasting" factor should be understood herein as being a compound or group of compounds being effective in the prevention or treatment of tissue and/or muscle wasting leading to an increased loss of body weight. It has been surprisingly found out that the anti-wasting effect of EPA is distinctly higher as compared to other ω-3 fatty acids such as docosahexaenoic acid (DHA). Therefore, the composition according to the present invention preferably comprises a source of EPA comprising not more than 20 wt%, preferably not more than 10 wt% and more preferably not more than 5 wt% of other components such as other fatty acids or derivatives thereof. In a particularly preferred embodiment EPA is employed as a pure EPA source containing about 100% EPA. According to the present invention EPA may be contained in the composition either as the free acids or as a derivative, such as its esters. Even if it is preferred that the pure EPA source is employed in the compositions of the present invention, the presence of other fatty acids or derivatives thereof is not excluded, as long as the improved anti-wasting effect of EPA is not affected. These fatty acids may comprise other ω-3 polyunsaturated fatty acids such as DHA, or ω-6 fatty acids. Suitable oil blends are commercially available and known to the skilled person.

In order to provide for an improved anti-wasting effect the composition according to the present invention comprises 0.1 to 10 g of EPA or a derivative thereof based on 100 kcal. As mentioned above, such an amount is remarkably high based on the low caloric value of the composition of the invention. Preferably the composition comprises 0.25 to 5 g, and most preferably 0.25 to 2 g EPA or a derivative thereof based on 100 kcal of the total composition. If the amount of EPA is below the above values, the anti-wasting effect of the composition is insufficient. If the amount of EPA is above said values, the anti-wasting effect again decreases due to an imbalance between the components of the composition. In order to provide for the maximum anti-wasting effect and cachexia/anorexia treatment effect EPA or its derivatives are administered to a patient in an amount of from 0.2 to 20 g per day, preferably from 0.5 to 10 g and more preferably from 0.5 to 4 g per day. In a particularly preferred embodiment 2 to 3 g EPA are administered per day, preferably as a pure EPA source. Furthermore, if the amount of EPA is above said values the caloric value of the total composition increases to a level rendering the composition unsuitable as a nutritional supplement which should have no or almost no influence on the total diet of the patient. In other words, if the amount of EPA exceeds the above-mentioned maximum values, the total composition tends to provide for a caloric value of more than 200 kcal, particularly of more than 150 kcal, being not preferable.

As a second essential component the composition according to the present invention comprises at least one phytochemical. The term "phytochemical" according to the present invention should be understood to comprise any compounds found in plants that have a beneficial effect on health or an active role in the amelioration of a disease, but that are not required for a normal functioning of the body. Phytochemicals are sometimes also referred to as phytonutrients. However, according to the present invention the term "phytochemical" or "phytonutrient" should be understood to not encompass terpenoids, such as carotenoids (including β-carotenes etc.), and vitamin E, vitamin C, or their derivatives, as these compounds according to the present invention are defined to fall under the group of further antioxidants described in more detail hereinbelow. Also the zinc, magnesium and selenium compounds should fall under the definition of antioxidants rather than of phytochemicals.

Suitable phytochemicals are known to the skilled person. According to the present invention they preferably comprise the group of polyphenolic compounds, glucosinolates, thiosulfonates, phytosterols, anthraquinones, capsaicin, and piperine. The polyphenolic compounds include flavonoids (anthocyanins, catechins, isoflavones, hesperetin, naringin, rutin, quercetin, silymarin, tangeretin, tannins, and punicalagin etc.) and phenolic acids (ellagic acid, chlorogenic acid, courmaric acid, phytic acid, ferulic acid, vanillin, cinnamic acid, and hydroxycinnamic acids etc.). Also other non-flavonoid polyphenolic compounds are comprised such as curcumin, resveratrol, and lignans.

Without being bound by any theory, the following is assumed. The activation of NFkB (nuclear factor-kappa B being a protein complex which is a transcription factor) is important for the induction of proteosome activity and protein degradation induced by TNF (tumor necrosis factor) and PIF (proteolysis inducing factor). Thus, agents capable in inhibiting NFkB induction have been evaluated in cachexia models. For example, the polyphenole resveratrol, which prevents activation of NFkB in skeletal muscle, was effective in the treatment of cachexia in an experimental tumor model system. Resveratrol, for example, also inhibited PIF-induced muscle loss and is therefore believed to interact beside EPA, glutamine and BCAA against the activation of the ATP ubiquitin-proteosme activated system.

The phytochemicals may be synthesized or extracted from natural sources by any suitable method known to those skilled in the art, particularly using food-grade solvents. Liquid and solid (e.g. granulate or powder form) extracts are suitable. Preferably the phytochemicals are employed according to the present invention as they occur in the respective natural source.

As a general rule, the phytochemical suitable according to the present invention may be derived from any plant, algae, or fungus. Preferably it is derived from chamomile, olive, red wine, green tea, and/or apple. In a particularly preferred embodiment it is contained as occurred within its natural source.

The phytochemicals comprised by the compositions according to the present invention preferably fulfill an ORAC value of 5,000 to 20,000 µmol/g extract. The determination of the ORAC value is in detail described in "Clinical Chemistry, 1995, Vol. 41, No 12" and provides a test for measuring the total antioxidative capacity of a substance or a nutrient. In a particularly preferred embodiment the ORAC value is within a range of from 7,000 to 15,000 µmol/g extract and more preferably within a range of from 10,000 to 15.000 µmol/g extract.

The phytochemical is contained in the composition according to the present invention in an amount of from 0.1 to 10 g based on 100 kcal of the total composition, preferably from 0.2 to 8 g, and more preferably from 0.5 to 3 g based on 100 kcal of the total composition. The composition according to the invention may also contain a mixture of phytochemicals. However, in such a case it is important to not exceed the total amount of 10 g of phytochemical mixture based on 100 kcal of the total composition in order to avoid an imbalance between the constituents of the nutritional composition according to the invention which imbalance would lead to a decreased anti-cachectic effect of the composition. Moreover, in case the amount of phytochemical is below the above-mentioned minimum values, the anti-cachectic effect of the composition is dramatically decreased.

The phytochemical or mixture of phytochemicals is administered to the patient in an amount of from 0.2 to 20 g per day, preferably from 0.4 to 16 g, and more preferably from 1 to 6 g per day. In case the daily dose of phytochemicals is outside the above ranges, the composition according to the invention fails to provide a sufficient anti-cachectic effect.

The nutritional compositions according to the present invention comprise as a third component one or more branched-chain amino acids (BCAA). The branched-chain amino acids according to the invention are intended to comprise amino acids that have a branch in the side chain and should include those having a carbon-carbon branch, i.e. valine (Val), leucine (Leu) and isoleucine (IIe). Even if valine, leucine and isoleucine are preferable and leucine is particularly preferable, also other types of branches containing amino acids are comprised according to the invention. The branched-chain amino acids may be comprised in neutral or salt form or in the form of a precursor or a derivative thereof.

Without being bound by theory, it is assumed that the anti-cachectic effect obtained by the branched-chain amino acids is based on their action as anti-serotonergic agents. In fact, anorexia and cachexia can be therapeutically treated by interfering with the neurochemical events downstream of cytokine activation. Serotonergic hypothalamic neuro-transmission may represent a suitable example. Synthesis of serotonin in the hypothalamus depends on the availability of its precursor, the amino acid tryptophan.

Such availability is reduced by the administration of branched chain amino acids (BCAA) because they compete with tryptophan for the same transport system located on the blood-brain barrier.

In a study, oral supplementation with BCAA improved anorexia and energy intake in anorectic cancer patients. Although not tested in lager clinical trials involving cancer patients, the prophagic effects of supplementation with BCAA have been confirmed in patients with uraemia and liver cirrhosis.

Another mechanism of action of BCAA could be that they act centrally to influence appetite and energy intake, while simultaneously inhibiting peripheral muscle wasting. It is postulated that melanocortin receptors are less activated as a result of reducing brain serotonergic activity via BCAA, leading to reduced peripheral muscle wasting.

Furthermore recent experimental data show that leucine, one of the three BCAA, has inhibitory effects on ATP-dependent ubiquitine activity.

The branched chain amino acids are further effective on muscle protein synthesis, while leucine has also been shown to inhibit expression of genes of the proteosome pathway in cachectic rats but not those with starvation. In addition, branched-chain amino acids may directly inhibit the protease "chymotrypsin" line activity.

The composition according to the invention preferably comprises 0.1 to 50 g, preferably 1 to 25 g, and more preferably 4 to 16 g, based on 100 kcal of the total composition, of branched-chain amino acids. In a particularly preferred embodiment the composition comprises valine, leucine, and isoleucine, preferably in the amounts of from 0.25 to 10 g leucine, 0.1 to 5 g isoleucine, and 0.1 to 5 g valine, based on 100 kcal of the total composition, and more preferably in the amounts of from 1 to 10 g leucine, 1 to 5 g isoleucine, and 1 to 5 g valine, based on 100 kcal of the total composition, respectively. Moreover, the branched-chain amino acids are preferably comprised in a ratio of Leu : Ile : Val of 25% to 75% : 15% to 35% : 15% to 35%. The branched-chain amino acids are preferably administered to a patient in a total amount of from 0.2 to 100 g per day, preferably from 2 to 50 g, and more preferably from 8 to 32 g per day. When the branched-chain amino acids are contained within the nutritional composition of the invention in the above-mentioned ranges, the anti-cachectic effect of the composition may be further improved. Particularly preferable leucine is administered within a range of from 6 to 50 g per day, isoleucine is administered from 3 to 25 g per day, and valine is administered from 3 to 25 g per day. Most preferable daily dosages are from 8 to 20 g Leu, 4 to 15 g Ile, and 4 to 15 g Val.

As a further component the composition of the invention comprises glutamine. Glutamine may be comprised in neutral or salt form or in the form of a precursor or a derivative thereof. Without being bound by any theory, glutamine is believed to be involved in the regulation of muscle protein synthesis and degradation. In septic rats, glutamine supplementation improves protein metabolism by decreasing protein breakdown and increasing protein synthesis in skeletal muscle and gut mucosa. Glutamine supplementation also increases protein synthesis and decreases protein breakdown in skeletal muscle of rats inoculated with AH109A hepatoma cells. In these studies, glutamine also diminishes body weight loss.

The use of Glutamine in cachexia treatment and prevention is effective in regard to improving muscle anabolism, slow down breakdown and inhibiting the ATP ubiquitin-proteosome system. Glutamine is also a very important substrate for macrophages, supporting the immune-system, and favoring anti-inflammatory cytokines, which could counteract the inflammatory status.

The composition of the invention preferably comprises from 0.25 to 50 g, more preferably from 0.25 to 10 g, and most preferably from 1 to 10 g of glutamine, based on 100 kcal of the total composition. When the amount of glutamine in the composition is within the above-mentioned ranges, the anti-cachectic effect of the composition may be further improved. Expressed on a daily dose, glutamine should be administered in a range of from 0.5 to 100 g, preferably from 0.5 to 50 g, more preferably from 8 to 50 g, and most preferably from 2 to 20 g per day. When administered within these ranges a maximum anti-cachectic effect may be obtained, without impacting the total diet of the patient by providing additional energy through the nutritional supplement according to the invention.

Additionally, the composition according to the invention may optionally contain further antioxidants commonly used in nutritional compositions and in particular in nutritional supplements. Preferably, the antioxidant is selected from the group consisting of terpenoids, vitamin E (comprising tocopherols and tocotrienols, both in α-, β-, γ-, and/or δ-forms), vitamin C, and selenium, zinc, and magnesium compounds as well as mixtures thereof. The terpenoids comprise carotenoid terpenoids and non-carotenoid terpenoids, while the carotenoid terpenoids (mixed carotenoids) comprising α-carotene, β-carotene, γ-carotene, lutein, lycopene, and/or zeaxanthin are particularly preferable. Typical non-carotenoid terpenoids are the saponins, terpeneol, and terpene limonoids. The above-mentioned compounds may be included into the nutritional compositions of the invention to generally combat oxidative stress and resultant genetic damage and slow the deterioration of collagen tissues. In general, antioxidants are believed to protect cells from cell damage (particularly by free radicals) leading to generation of cancerous cells. Furthermore, antioxidants generally protect tissue, particular vascular and capillary tissue, from deterioration, which is believed to inhibit metastases and cancer recurrence. Also in the cachexia syndrome a high level of oxidative stress is believed to exist.

Several mechanisms may lead to oxidative stress in the cachexia syndrome: without being bound by any theory it is believed that the body redox systems, which include antioxidant enzymes and low molecular weight antioxidants, may be unregulated in cachectic patients, and that this imbalance might enhance disease progression. Several evidence has been provided about the mechanisms linking oxidative stress and cancer cachexia, and clinically significant oxidative stress takes place in advanced cancer patients for example. Both cachexia syndrome and oxidative stress alone or in combination are highly predictive of clinical outcome and survival. Therefore a supplementation in order to avoid oxidative stress in these patients which in turn might also conduct to a higher inflammatory state-inducing tissue wasting is considered reasonable according to a preferred embodiment of the present invention.

In a particularly preferred embodiment the composition comprises 0.25 to 10 mg and preferably 1 to 10 mg mixed carotenoids based on 100 kcal of the total composition. More particular, the compostion preferably comprises 0.1 to 10 mg and more preferably 0.5 to 5 mg β-carotene. Generally, in 5 mg mixed carotenoids (cf. Table 1 below) about 2 mg β-carotene, 1.35 mg α-carotene, 0.65 mg lutein, 1 mg lycopene, and 0.1 mg zeaxanthin and γ-carotene are contained. In a further preferred embodiment the composition additionally comprises 3 to 300 mg, preferably 10 to 150 mg vitamin E; 10 to 500 mg, preferably 15 to 500 mg vitamin C; and 3 to 300 µg, preferably 15 to 150µg selenium, based on 100 kcal of the total composition, respectively. Expressed on a daily dose, 0.5 to 40 mg, preferably 10 to 40 mg, and more preferably 2 to 20 mg mixed carotenoids; 6 to 600 mg, preferably 20 to 300 mg vitamin E; 20 to 2000 mg, preferably 200 to 1000 mg vitamin C; and 6 to 750 µg, preferably 50 to 300 µg selenium are preferably administered to a patient in order to further improve the anti-cachectic activity of the nutritional composition of the invention.

As further optional components, the nutritional composition may comprise nutritional compounds commonly employed, such as stabilizers, colorants, flavors, pH adjusting agents, further vitamins (such as vitamins A, D, K, folic acid, thiamine, riboflavin, vitamin B₆, vitamin B₁₂, niacin, etc), minerals, sweeteners, anti-foam agents, and fillers. The compositions may also additionally comprise macronutrients, such as fats, carbohydrates, proteins, and fiber components. However, these additional components are preferably contained to such an extent not exceeding a coloric value of 400 kcal of the composition based on a daily dose to be administered, preferably of 300 kcal and more preferably of 200 kcal. As mentioned above, in preferred embodiments of the present invention the total caloric value of the compositions is basically provided by EPA, BCAA and glutamine. In particular, the amount of carbohydrates should be as low as possible, i.e. below 10% wt-%, preferably below 5 % wt-%, and more preferably 0% wt-%. Thus, carbohydrates should be no (or almost no) caloric carriers in accordance with the present invention.

The nutritional composition according to the invention is for use as a medicament, preferably as a nutritional supplement, for the treatment and/or prevention of cachexia and/or anorexia. It is particularly useful in the prevention of cachexia at a very early preclinical stage. It has surprisingly been found by the present inventors that there is existing a synergistic effect between the components of the composition and in particular between eicosapentaenoic acid (EPA) and the phytochemicals. Moreover, the additional presence of branched-chain amino acids (BCAA), in particular of leucine, isoleucine, and valine, as well as the presence of glutamine further improves the anti-cachectic/anti-anorectic effects and anti-wasting effects (muscle and tissue wasting) and leads to an increase of these effects being distinctly above the level to be expected from the sum of effects achieved by the respective single components alone. The present invention has been accomplished on the basis of such an unexpected finding. In view thereof, it has become possible according to the present invention for the first time to combine a treatment adapted to the underlying pathophysiology of the cachexia syndrome in order to prevent the onset of cachexia by itself. The components of the composition act on the different sites of cachexia onset as muscle and fat tissue, brain (hypothalamus) for appetite regulation and the hormonal system.

Therefore the use of the synergistic acting pharmaconutrients according to the invention renders it possible to counteract the cachexia inducing metabolic changes. Without being bound by any theory, it is assumed that this synergistic effect is based on the action and positive influence of the compounds of the inventive composition at different sites of the ATP ubiquitin-dependent proteolytic pathway.

The nutritional composition usually is present in a dry form as a powder or granules. Before administration it is dissolved in a suitable amount of water. For the administration the respective components are either present in a single composition or in separate vehicles. The composition is preferably administered orally. However, also the enteral and intravenous administration is possible. The volume of the aqueous solution of the composition to be administered can be easily set to a reasonable value and usually is within a range of from 100 to 150 ml per serving. It is particularly suitable to administer the composition several times per day, most preferably twice a day.

The nutritional composition may be prepared by using methods known to the skilled person. In a preferred embodiment the respective components are simply mixed with each other in any order. However, it is also possible to encapsulate the single substances by known and commonly used encapsulation processes before mixing them. The encapsulation is usually done in order to improve the storage stability of the compositions in dry form.

The invention will now be explained in more detail by making reference to the following example.

### Example 1

A nutritional composition according to the invention is prepared by mixing the following components in the respective amounts as indicated in Table 1. No encapsulation is carried out prior to the mixing. The composition as indicated in Table 1 provides for a caloric value of about 100 kcal. The amounts of the respective component correspond to 50% of the recommended daily dose so that the indicated composition should be administered twice a day. The caloric value can be determined by a suitable method known in the art.

**Table 1**

| **Component** | **Amount** |
|---|---|
| Eicosapentaenoic acid (EPA) | 1 g |
| Red wine extract | 1,5 g |
| Valine | 2,5 g |
| Leucine | 5g |
| Isoleucine | 2,5 g |
| Glutamine | 5 g |
| mixed carotenoids | 5 mg |
| Vitamin E | 50 mg |
| Vitamin C | 250 mg |
| Selenium | 50 µg |

The above-mentioned composition is packaged into sachets providing for a caloric value of about 100 kcal.

## Claims

1. A nutritional composition comprising:
(a) from 0.1 to 10 g of eicosapentaenoic acid or a derivative thereof based on 100 kcal of the total composition;
(b) from 0.1 to 10 g of a phytochemical or a mixture thereof, other than terpenoids, vitamin E, and vitamin C, or their derivatives, based on 100 kcal of the total composition;
(c) from 0.1 to 50 g based on 100 kcal of the total composition of one or more branched-chain amino acids selected from the group consisting of leucine (Leu), isoleucine (Ile), and valine (Val), in neutral or salt form or a precursor or derivative thereof; and
(d) from 0.25 to 50 g of glutamine in neutral or salt form or a precursor or derivative thereof based on 100 kcal of the total composition.

2. The composition according to claim 1, wherein the branched-chain amino acids are comprised in a ratio of Leu : Ile : Val of 25% to 75% : 15% to 35% : 15% to 35%.

3. The composition according to any one of claims 1 and/or 2, further comprising:
(e) one or more antioxidants selected from the group consisting of mixed carotenoids, vitamin E, vitamin C, zinc, magnesium, and selenium.

4. The composition according to claim 3, comprising 0.25 to 10 mg, preferably 1 to 10 mg mixed carotenoids; 3 to 300 mg, preferably 10 to 150 mg vitamin E; 10 to 500 mg, preferably 15 to 500 mg vitamin C; and 3 to 300 µg, preferably 15 to 150 µg selenium, based on 100 kcal of the total composition.

5. The composition according to any one of claims 1 to 4, wherein the phytochemical fulfills an ORAC value of from 5,000 to 20,000 µmol/g extract, preferably from 10,000 to 15,000 µmol/g extract.

6. The composition according to any one of claims 1 to 5, wherein the phytochemical is derived from camomile, olive, red wine, green tea, and/or apple, and is preferably contained as occured within its natural source.

7. The composition according to any one of claims 1 to 6, comprising:
(a) from 0.1 to 10 g of eicosapentaenoic acid or a derivative thereof based on 100 kcal of the total composition;
(b) from 0.1 to 10 g of a phytochemical or a mixture thereof, other than terpenoids, vitamin E, and vitamin C, or their derivatives, based on 100 kcal of the total composition;
(c) from 0.25 to 10 g leucine (Leu), from 0.1 to 5 g isoleucine (Ile), and from 0.1 to 5 g valine (Val), in neutral or salt form or a precursor or derivative thereof based on 100 kcal of the total composition, respectively;
(d) from 0.25 to 10 g of glutamine in neutral or salt form or a precursor or derivative thereof based on 100 kcal of the total composition; and
(e) 0.25 to 10 mg mixed carotenoids, 3 to 300 mg vitamin E, 10 to 500 mg vitamin C, and 3 to 300 µg selenium based on 100 kcal of the total composition.

8. A nutritional composition comprising:
(a) from 0.2 to 20 g of eicosapentaenoic acid or a derivative thereof per daily dose;
(b) from 0.2 to 20 g of a phytochemical or a mixture thereof, other than terpenoids, vitamin E, and vitamin C, or their derivatives, per daily dose;
(c) from 0.2 to 100 g of one or more branched-chain amino acids selected from the group consisting of leucine (Leu), isoleucine (Ile), and valine (Val), in neutral or salt form or a precursor or derivative thereof per daily dose;
(d) from 0.5 to 100 g of glutamine in neutral or salt form or a precursor or derivative thereof per daily dose; and
(e) optionally, 10 to 40 mg mixed carotenoids, 40 to 600 mg vitamin E, 200 to 2000 mg vitamin C, and 50 to 750 µg selenium per daily dose.

9. The composition according to any on of claims 1 to 8 for use as a medicament, preferably as a nutritional supplement.

10. The composition according to any one of claims 1 to 8 for use in the treatment and/or prevention of cachexia and/or anorexia.

11. A nutritional composition comprising:
(a) eicosapentaenoic acid or a derivative thereof;
(b) at least one phytochemical, other than terpenoids, vitamin E, and vitamin C, or their derivatives;
(c) one or more branched-chain amino acids selected from the group consisting of leucine (Leu), isoleucine (Ile), and valine (Val), in neutral or salt form or a precursor or derivative thereof;
(d) glutamine in neutral or salt form or a precursor or derivative thereof; and
(e) optionally, mixed carotenoids, vitamin E, vitamin C, and selenium,
for use in the treatment and/or prevention of cachexia and/or anorexia,
wherein the dosages to be administered are from 0.2 to 20 g/day eicosapentaenoic acid or a derivative thereof; from 0.2 to 20 g/day phytochemicals; from 6 to 50 g/day leucine; from 3 to 25 g/day isoleucine; from 3 to 25 g/day valine; from 8 to 50 g/day glutamine; and, if present in the composition, 10 to 40 mg/day mixed carotenoids, 40 to 600 mg/day vitamin E, 200 to 2000 mg/day vitamin C, and 50 to 750 µg/day selenium.

12. The composition according to any one of claims 10 and/or 11, wherein the total caloric value supplied by the composition to the patient per day is not more than 400 kcal, preferably not more than 300 kcal, and more preferably not more than 200 kcal.

13. Medicament, preferably a nutritional supplement, for use in the treatment and/or prevention of cachexia and/or anorexia comprising the composition according to any one of claims 1 to 8 and a pharmaceutically acceptable excipient.

14. Sachet comprising the composition according to any one of claims 1 to 7, which provides for a caloric value of not more than 200 kcal, preferably not more than 150 kcal.

## Patentansprüche

1. Nahrungszusammensetzung, umfassend:
(a) 0,1 bis 10 g Eicosapentaensäure oder eines Derivats davon auf der Basis von 100 kcal der Gesamtzusammensetzung;
(b) 0,1 bis 10 g eines sekundären Pflanzenstoffs oder einer Mischung davon, außer Terpenoide, Vitamin E und Vitamin C, oder deren Derivate, auf der Basis von 100 kcal der Gesamtzusammensetzung;
(c) 0,1 bis 50 g auf der Basis von 100 kcal der Gesamtzusammensetzung einer oder mehrerer verzweigtkettiger Aminosäuren, ausgewählt aus der Gruppe, bestehend aus Leucin (Leu), Isoleucin (Ile) und Valin (Val), in neutraler Form oder Salzform, oder einer Vorstufe oder eines Derivats davon; und
(d) 0,25 bis 50 g Glutamin in neutraler Form oder Salzform, oder einer Vorstufe oder eines Derivats davon, auf der Basis von 100 kcal der Gesamtzusammensetzung.

2. Zusammensetzung gemäß Anspruch 1, wobei die verzweigtkettigen Aminosäuren in einem Verhältnis Leu : Ile : Val von 25 % bis 75 % : 15 % bis 35 % : 15 % bis 35 % umfasst sind.

3. Zusammensetzung gemäß einem der Ansprüche 1 oder 2 des Weiteren umfassend:
(e) ein oder mehrere Antioxidantien, ausgewählt aus der Gruppe, bestehend aus gemischten Carotinoiden, Vitamin E, Vitamin C, Zink, Magnesium und Selen.

4. Zusammensetzung gemäß Anspruch 3, umfassend 0,25 bis 10 mg, bevorzugt 1 bis 10 mg gemischte Carotinoide; 3 bis 300 mg, bevorzugt 10 bis 150 mg Vitamin E; 10 bis 500 mg, bevorzugt 15 bis 500 mg Vitamin C; und 3 bis 300 µg, bevorzugt 15 bis 150 µg Selen auf der Basis von 100 kcal der Gesamtzusammensetzung.

5. Zusammensetzung gemäß einem der Ansprüche 1 bis 4, wobei der sekundäre Pflanzenstoff einen ORAC-Wert von 5.000 bis 20.000 µmol/g Extrakt, bevorzugt von 10.000 bis 15.000 µmol/g Extrakt erfüllt.

6. Zusammensetzung gemäß einem der Ansprüche 1 bis 5, wobei der sekundäre Pflanzenstoff aus Kamille, Olive, Rotwein, grünem Tee und/oder Apfel stammt und bevorzugt wie in seiner natürlichen Herkunft vorkommend enthalten ist.

7. Zusammensetzung gemäß einem der Ansprüche 1 bis 6, umfassend:
(a) 0,1 bis 10 g Eicosapentaensäure oder eines Derivats davon auf der Basis von 100 kcal der Gesamtzusammensetzung;
(b) 0,1 bis 10 g eines sekundären Pflanzenstoffs oder einer Mischung davon, außer Terpenoide, Vitamin E und Vitamin C, oder deren Derivate, auf der Basis von 100 kcal der Gesamtzusammensetzung;
(c) 0,25 bis 10 g Leucin (Leu), 0,1 bis 5 g Isoleucin (Ile) und 0,1 bis 5 g Valin (Val), in neutraler Form oder Salzform, oder einer Vorstufe oder eines Derivats davon, jeweils auf der Basis von 100 kcal der Gesamtzusammensetzung;
(d) 0,25 bis 10 g Glutamin in neutraler Form oder Salzform oder einer Vorstufe oder eines Derivats davon, auf der Basis von 100 kcal der Gesamtzusammensetzung; und
(e) 0,25 bis 10 mg gemischte Carotinoide, 3 bis 300 mg Vitamin E, 10 bis 500 mg Vitamin C und 3 bis 300 µg Selen, auf der Basis von 100 kcal der Gesamtzusammensetzung.

8. Nahrungszusammensetzung, umfassend:
(a) 0,2 bis 20 g Eicosapentaensäure oder eines Derivats davon, pro Tagesdosis;
(b) 0,2 bis 20 g eines sekundären Pflanzenstoffs oder einer Mischung davon, außer Terpenoide, Vitamin E und Vitamin C, oder deren Derivate, pro Tagesdosis;
(c) 0,2 bis 100 g einer oder mehrerer verzweigtkettiger Aminosäuren, ausgewählt aus der Gruppe, bestehend aus Leucin (Leu), Isoleucin (Ile) und Valin (Val), in neutraler Form oder Salzform, oder einer Vorstufe oder eines Derivats davon, pro Tagesdosis;
(d) 0,5 bis 100 g Glutamin in neutraler Form oder Salzform, oder einer Vorstufe oder eines Derivats davon, pro Tagesdosis; und
(e) wahlweise 10 bis 40 mg gemischte Carotinoide, 40 bis 600 mg Vitamin E, 200 bis 2.000 mg Vitamin C und 50 bis 750 µg Selen, pro Tagesdosis.

9. Zusammensetzung gemäß einem der Ansprüche 1 bis 8 zur Verwendung als Medikament, bevorzugt als Nahrungsergänzungsmittel.

10. Zusammensetzung gemäß einem der Ansprüche 1 bis 8 zur Verwendung in der Behandlung und/oder Prävention von Kachexie und/oder Anorexie.

11. Nahrungszusammensetzung, umfassend:
(a) Eicosapentaensäure oder ein Derivat davon;
(b) mindestens einen sekundären Pflanzenstoff, außer Terpenoide, Vitamin E und Vitamin C, oder deren Derivate;
(c) eine oder mehrere verzweigtkettige Aminosäuren, ausgewählt aus der Gruppe, bestehend aus Leucin (Leu), Isoleucin (Ile) und Valin (Val), in neutraler Form oder Salzform oder einer Vorstufe oder eines Derivats davon;
(d) Glutamin in neutraler oder Salzform oder eine Vorstufe oder Derivat davon; und
(e) wahlweise gemischte Carotinoide, Vitamin E, Vitamin C und Selen, zur Verwendung in der Behandlung und/oder Prävention von Kachexie und/oder Anorexie, wobei die zu verabreichenden Dosen 0,2 bis 20 g/Tag Eicosapentaensäure oder eines Derivats davon; 0,2 bis 20 g/Tag sekundäre Pflanzenstoffe; 6 bis 50 g /Tag Leucin; 3 bis 25 g/Tag Isoleucin; 3 bis 25 g/Tag Valin; 8 bis 50 g/Tag Glutamin; und, falls in der Zusammensetzung vorhanden, 10 bis 40 mg/Tag gemischte Carotinoide, 40 bis 600 mg/Tag Vitamin E, 200 bis 2.000 mg/Tag Vitamin C und 50 bis 750 µg/Tag Selen sind.

12. Zusammensetzung gemäß einem der Ansprüche 10 und/oder 11, wobei der Gesamtwärmewert, der dem Patienten pro Tag durch die Zusammensetzung bereitgestellt wird, nicht mehr als 400 kcal, bevorzugt nicht mehr als 300 kcal und am bevorzugtesten nicht mehr als 200 kcal beträgt.

13. Medikament, bevorzugt Nahrungsergänzungsmittel zur Verwendung in der Behandlung und/oder Prävention von Kachexie und/oder Anorexie, umfassend die Zusammensetzung gemäß einem der Ansprüche 1 bis 8 und einen pharmazeutisch unbedenklichen Hilfsstoff.

14. Sachet, umfassend die Zusammensetzung gemäß einem der Ansprüche 1 bis 7, die einen Wärmewert von nicht mehr als 200 kcal, bevorzugt nicht mehr als 150 kcal bereitstellt.

## Revendications

1. Composition nutritionnelle comprenant:
(a) de 0, 1 à 10 g d'acide éicosapentaénoïque ou d'un dérivé de celui-ci en se basant sur 100 kcal de la composition totale ;
(b) de 0,1 à 10 g d'un produit phytochimique ou d'un mélange de produits phytochimiques, autres que des terpénoïdes, de la vitamine B, et de la vitamine C, ou leurs dérivés, en se basant sur 100 kcal de la composition totale ;
(c) de 0,1 à 50 g en se basant sur 100 kcal de la composition totale d'un ou de plusieurs acides aminés à chaîne ramifiée choisis dans le groupe constitué par la leucine (Leu), l'isoleucine (Ile), et la valine (Val), sous une forme neutre ou sous la forme d'un sel ou un précurseur ou un dérivé de celles-ci ; et
(d) de 0,25 à 50 g de glutamine sous une forme neutre ou sous la forme d'un sel ou un précurseur ou un dérivé de celle-ci en se basant sur 100 kcal de la composition totale.

2. Composition selon la revendication 1, dans laquelle les acides aminés à chaîne ramifiée sont compris en un rapport de Leu/Ile/Val de 25 % à 75 %/15 % à 35 %/15 % à 35 %.

3. Composition selon l'une quelconque des revendications 1 et/ou 2, comprenant en outre :
(e) un ou plusieurs antioxydants choisis dans le groupe constitué par les caroténoïdes mixtes, la vitamine E, la vitamine C, le zinc, le magnésium, et le sélénium.

4. Composition selon la revendication 3, qui comprend de 0,25 à 10 mg, de préférence de 1 à 10 mg de caroténoïdes mixtes ; de 3 à 300 mg, de préférence de 10 à 150 mg de vitamine E ; de 10 à 500 mg, de préférence de 15 à 500 mg de vitamine C ; et de 3 à 300 µg ; de préférence de 15 à 150 µg de sélénium, en se basant sur 100 kcal de la composition totale.

5. Composition selon l'une quelconque des revendications 1 à 4, dans laquelle le produit phytochimique a une valeur ORAC de 5 000 à 20 000 µmol/g d'extrait, de préférence de 10 000 à 15 000 µmol/g d'extrait.

6. Composition selon l'une quelconque des revendications 1 à 5, dans laquelle le produit phytochimique est dérivé de camomille, d'olive, de vin rouge, de thé vert, et/ou de pomme, et est de préférence contenu tel qu'il se trouve dans sa source naturelle.

7. Composition selon l'une quelconque des revendications 1 à 6, qui comprend :
(a) de 0,1 à 10 g d'acide éicosapentaénoïque ou d'un dérivé de celui-ci en se basant sur 100 kcal de la composition totale ;
(b) de 0,1 à 10 g d'un produit phytochimique ou d'un mélange de produits phytochimiques, autres que des terpénoïdes, la vitamine E, et la vitamine C, ou leurs dérivés, en se basant sur 100 kcal de la composition totale ;
(c) de 0,25 à 10 g de leucine (Leu), de 0,1 à 5 g d'isoleucine (Ile), et de 0,1 à 5 g de valine (Val), sous une forme neutre ou sous la forme d'un sel ou un précurseur ou un dérivé de celles-ci en se basant sur 100 kcal de la composition totale, respectivement ;
(d) de 0,25 à 10 g de glutamine sous une forme neutre ou sous la forme d'un sel ou un précurseur ou un dérivé de celle-ci en se basant sur 100 kcal de la composition totale ; et
(e) de 0,25 à 10 mg de caroténoïdes mixtes, de 3 à 300 mg de vitamine E, de 10 à 500 mg de vitamine C, et de 3 à 300 µg de sélénium en se basant sur 100 kcal de la composition totale.

8. Composition nutritionnelle comprenant :
(a) de 0,2 à 20 g d'acide éicosapentaénoïque ou d'un dérivé de celui-ci par dose quotidienne ;
(b) de 0,2 à 20 g d'un produit phytochimique ou d'un mélange de produits phytochimiques, autres que des terpénoïdes, la vitamine E, et la vitamine C, ou leurs dérivés, par dose quotidienne ;
(c) de 0,2 à 100 g d'un ou de plusieurs acides aminés à chaîne ramifiée choisis dans le groupe constitué par la leucine (Leu), l'isoleucine (Ile), et la valine (Val), sous la forme d'un sel ou sous une forme neutre ou un précurseur ou un dérivé de celles-ci par dose quotidienne ;
(d) de 0,5 à 100 g de glutamine sous une forme neutre ou sous la forme d'un sel ou un précurseur ou un dérivé de celle-ci par dose quotidienne ; et
(e) facultativement, de 10 à 40 mg de caroténoïdes mixtes, de 40 à 600 mg de vitamine E, de 200 à 2 000 mg de vitamine C, et de 50 à 750 µg de sélénium par dose quotidienne.

9. Composition selon l'une quelconque des revendications 1 à 8, destinée à être utilisée comme un médicament, de préférence comme un supplément nutritionnel.

10. Composition selon l'une quelconque des revendications 1 à 8 destinée à être utilisée dans le traitement et/ou la prévention de la cachexie et/ou de l'anorexie.

11. Composition nutritionnelle comprenant :
(a) de l'acide éicosapentaénoïque ou un dérivé de celui-ci ;
(b) au moins un produit phytochimique, autre que des terpénoïdes, la vitamine E, et la vitamine C, ou leurs dérivés ;
(c) un ou de plusieurs acides aminés à chaîne ramifiée choisis dans le groupe constitué par la leucine (Leu), l'isoleucine (Ile), et la valine (Val), sous une forme neutre ou sous la forme d'un sel ou un précurseur ou un dérivé de celles-ci ;
(d) de la glutamine sous une forme neutre ou sous la forme d'un sel ou un précurseur ou un dérivé de celle-ci ; et
(e) facultativement, des caroténoïdes mixtes, de la vitamine E, de la vitamine C, et du sélénium,
destinée à être utilisée dans le traitement et/ou la prévention de la cachexie et/ou de l'anorexie,
les doses à administrer étant de 0,2 à 20 g/jour d'acide éicosapentaénoïque ou d'un dérivé de celui-ci ; de 0,2 à 20 g/jour de produits phytochimiques ; de 6 à 50 g/jour de leucine ; de 3 à 25 g/jour d'isoleucine ; de 3 à 25 g/jour de valine ; de 8 à 50 g/jour de glutamine ; et s'il y en a dans la composition, de 10 à 40 mg/jour de caroténoïdes mixtes, de 40 à 600 mg/jour de vitamine E, de 200 à 2 000 mg/jour de vitamine C, et de 50 à 750 µg/jour de sélénium.

12. Composition selon l'une quelconque des revendications 10 et/ou 11, la valeur calorique totale fournie quotidiennement par la composition au patient n'excédant pas 400 kcal, de préférence n'excédant pas 300 kcal, et idéalement n'excédant pas 200 kcal.

13. Médicament, de préférence un supplément nutritionnel, destiné à être utilisé dans le traitement et/ou la prévention de la cachexie et/ou de l'anorexie qui comprend la composition selon l'une quelconque des revendications 1 à 8 et un excipient pharmaceutiquement acceptable.

14. Sachet comprenant la composition selon l'une quelconque des revendications 1 à 7, qui fournit une valeur calorique n'excédant pas 200 kcal, de préférence n'excédant pas 150 kcal.
